# EUROPEAN PATENT SPECIFICATION

(11) **EP 1 768 682 B1**
(45) Date of publication and mention of the grant of the patent: **08.10.2008**
(21) Application number: 05760934.9
(22) Date of filing: 21.06.2005
(51) Int. Cl.: A61K 36/00, A61K 36/48, A61K 36/61, A61P 9/00

(54) **PRODUCT OF VEGETAL ORIGIN COMPRISING PROANTHOCYANIDINES AND ITS PREPARATION PROCESS**
PRODUKT PFLANZLICHEN URSPRUNGS, DAS PROANTHOCYANE ENTHÄLT, SOWIE VERFAHREN ZU SEINER HERSTELLUNG
PRODUIT D'ORIGINE VEGETALE COMPRENANT DES PROANTHOCYANIDINES ET SON PROCEDE DE PREPARATION

(30) Priority: 23.06.2004 ES 200401626
(43) Date of publication of application: 04.04.2007
(73) Proprietor: Investigacion Y Nutricion, S.L., 28023 Madrid (ES)
(72) Inventor: RUIZ-ROSO CALVO DE MORA, Baltasar, E-28220 Majadahonda (ES); REQUEJO MARCOS, Ana María, E-28023 Madrid (ES); PEREZ-OLLEROS CONDE, Lourdes, E-28028 Madrid (ES); HOLGUIN HUESO, José Antonio, Exxentia, S.A., E-28031 Madrid (ES)
(74) Representative: Barlocci, Anna
(86) International application number: PCT/EP2005/052877
(87) International publication number: WO 2006/000551

(56) References cited:
- EP-A- 0 713 706
- WO-A-90/13304
- WO-A-20/04014150
- WURSCH P ET AL: "TANNIN GRANULES FROM RIPE CAROB CERATONIA-SILIQUA POD" LEBENSMITTEL-WISSENSCHAFT AND TECHNOLOGIE, vol. 17, no. 6, 1984, pages 351-354, XP009054191 ISSN: 0023-6438
- MARAKIS S: "CARBO BEAN IN FOOD AND FEED: CURRENT STATUS AND FUTURE POTENTIALS - A CRITICAL APPRAISAL" JOURNAL OF FOOD SCIENCE AND TECHNOLOGY, ASSOCIATION OF FOOD SCIENTISTS AND TECHNOLOGISTS, US, vol. 33, no. 5, 1996, pages 365-383, XP009015221 ISSN: 0022-1155

## Description

This invention relates to the field of the pharmaceutical, dietary and food industries, in particular to products related with the control of risk factors of cardiovascular diseases.

### BACKGROUND ART

High level of cholesterol in blood is a risk factor of cardiovascular diseases. Different studies have demonstrated that populations which consume diets rich in fiber have lower quantities of cholesterol and LDL in blood than populations which consume less fiber (cf. M.H. Davidson et al., J. Am. Coll. Cardiol. 1998, vol. 31 suppl. pp. 1114-5). The beneficial effects of fiber are also known for the protection of the intestinal barrier integrity and for the cancer of colon prevention.

Nevertheless, nowadays, the therapeutic application of dietary fiber supplements available in the market is very problematic, mainly due to these natural fibers are mixtures of substances with a very variable and heterogeneous chemical composition with the only common property that they are components of food which are not attacked by human digestive enzymes. This is the reason why is difficult to define their active ingredients and quantify its dose.

Dietary fibers contain pharmacologically active compounds, but they mainly contain products with little or no activity, such as celluloses or hemicelluloses. Minority components of the dietary fiber are polyphenols. The term polyphenol comprises a great number of secondary metabolites of vegetables, which are structurally characterized by having aromatic rings with hydroxyl groups. In plants, polyphenols can be found free or associated to components of the cellular wall. This localization together with their molecular weight, allows the differentiation between soluble and insoluble polyphenols. Insoluble polyphenols have a bigger molecular weight and have the beneficial effects on health above indicated.

Nevertheless, insoluble polyphenolic compounds are present in a very little amount in fiber of the most commonly consumed vegetables or in commercial dietary supplements of fiber. Besides, most of these insoluble polyphenols are not usable at their natural state in the necessary doses for a chronic treatment of degenerative diseases because they have a strong astringent and anti-nutritional effect, due to their inhibition activity of the digestive enzymes by forming complexes with proteins.

One of the dietary fibers with more polyphenols content is carob bean fiber, from fruit of carob tree (Ceratonia Siliqua). The first commercial fiber with a high polyphenols content is the one called "carob fiber", of C.G.A. S.A. (cf. ES 2.060.543). Another carob bean fiber is described in the patent document ES 2.204.301. Both fibers have a polyphenols content of approximately 50% of the dry weight. These fibers reduce cholesterol levels in rats but extrapolation to humans of the doses used in studies with animals would mean amounts between 25 and 50 g/day, very high doses for its therapeutic use.

It has been tried to increase the insoluble polyphenols content in a dietary fiber. The most outstanding example is a carob bean fiber containing near a 90% of insoluble polyphenols which have been thermally modified in order to eliminate their astringent and anti-nutritional effects (cf. ES 2.187.356).

### SUMMARY OF THE INVENTION

It is desirable to provide dietary fibers with a high percentage of insoluble polyphenols with the aim to produce a maximum hypocholesterolaemic effect at acceptable doses for human consume and with low astringent and anti-nutritional effects. It is also desirable to provide industrially viable processes for its preparation.

Thus, the invention provides a new product with a high percentage of polyphenols with high molecular weight. The invention also provides industrial processes for its preparation.

In a first aspect the invention provides a product of vegetal origin that comprises a 65-97% percent by weight of proanthocyanidines with molecular weight superior to 6000 daltons, more than a 50% percent by weight of said proanthocyanidines have a molecular weight superior to 30000 daltons. In a particular embodiment of the invention, the percent by weight of the proanthocyanidines with molecular weight superior to 6000 daltons is 90-97%. Particularly, the percent by weight of the proanthocyanidines with molecular weight superior to 30000 daltons versus the proanthocyanidines with molecular weight superior to 6000 daltons is higher than 60%.

Even though there are some differences, in this description the term proanthocyanidine is considered as a synonym of the term condensed tannin, being both chemically polymerized derivatives of flavonoids, mainly of flavan-3-ol. Then, they are a type of high molecular weight polyphenols.

In another particular embodiment of the invention, with the intention of reducing possible astringent and anti-nutritional effects, the product of vegetal origin indicated above further comprises a 0-2% percent by weight, and particularly a 0-1%, of cold water-soluble polyphenols.

The composition of the product of vegetal origin, depending on the vegetal variety used and the specific conditions of the preparation process is as follows:
- Humidity: 0-10%
- Minerals: 2-10%
- Proanthocyanidines with molecular weight superior to 6000: 65-97% (proanthocyanidines with molecular weight superior to 30000: more than a 50% of said proanthocyanidines)
- Water-soluble polyphenols: 0-2%.
- Celluloses and hemicelluloses: 0-5%.
- Lignin: 0-10%
- Oligosaccharides: 0-5%
- Proteins: 0-5%

The product of vegetal origin of the present invention is a brown amorphous powder. It is hardly soluble in water and in low molecular weight alcohols at room temperature. The solubility rises when the temperature is close to the boiling point of the solvent or of the solvents mixture. Their solutions give a positive reaction of polyphenols with Folin-Ciocalteau reactive and a precipitation with lead salts. The product of vegetal origin of the invention is a product mainly indigestible and non-absorbable in the intestinal tract.

Due to the chemical structure of the proanthocyanidines present in the product of the invention, they produce a hydrophobic-type interaction with cholesterol and bile salts, which thus, are eliminated through feces as complexes with said proanthocyanidines. Therefore, the enterohepatic cycle of cholesterol and bile salts is interrupted and thus, cholesterol diminishes in the organism. Due to the enrichment in proanthocyanidines with high molecular weight, the product of vegetal origin of the invention produces a maximum hipocholesterolaemic effect at acceptable doses for human consume, usable in a pharmaceutical form, and with low astringent and anti-nutritional effects.

In a particular embodiment of the invention, the product is obtained from dicotyledons. It can be obtained from the whole plant or from parts of it, including fruits in ripping state and preferably dried. Plants and their fruits are collected in ripping state, generally at the end of summer and are left to dry up in the shade at room temperature and at low relative humidity conditions. Examples of appropriate vegetal varieties to be used for the preparation of the product of the invention are lucerne (Medicago sativa), field eryngo (Eryngium campestre), holm oak bark (Quercus ilex), pomegranate tree (Punica granatum) and quebracho (Aspindosperma guebracho-bianco). Examples of fruits that can be used are carob bean (Ceratonia siliqua), fruit from walnut tree (Juglans-regia), olive (Olea europea), blueberry (Vaccinuium myrtillus), pomegranate (Punica granatum) and apple (Pyrus malus). Preferably, the product of the invention is obtained from pomegranate or from carob bean pulp.

In a second aspect, the invention provides a preparation process of the product of vegetal origin described above, which comprises the steps of: (a) performing a wash of the crushed vegetal material with a liquid selected from the group consisting of water between room temperature and 60 °C; a mixture of water and a C₁-C₃ alcohol between 20:80 and 5:95 (v/v) at room temperature; and a mixture of water and acetone between 20:80 and 40:60 (v/v) at room temperature; and separating the solid and the liquid resulting from the wash, as many times as needed to get the washing liquid sweeping along less than 50 g of material per liter of washing liquid; (b) performing an extraction from the solid obtained in step (a) at room or pressurized pressure, in conditions selected from the group consisting of water between 80 °C and its boiling point; a C₁-C₃ alcohol between 40 °C and its boiling point; a mixture of water and a glycol between 80 °C and its boiling point; dimethylformamide between 80 °C and its boiling point; and dimethylsulphoxide between 80-150 °C, and separating the solid and the liquid resulting from the extraction keeping a temperature higher than 40 °C; (c) chilling the liquid obtained in step (b) and collecting the resulting precipitate, optionally evaporating part of the solvent before chilling; (d) drying the precipitate obtained in step (c) until a solvent content lower than 10% percent by weight; (e) subjecting the product obtained in step (d) to 120-180 °C for at least 3 minutes; and (f) chilling the product obtained in step (e).

Preferably, the starting vegetal material for this process is as commercial dehydrated and crushed pulp. When carob bean used, pulp without seeds will be preferred, but depending on the fruit or the vegetal variety, seeds could be included. Starting from no-commercial vegetal material, before the extraction process, it has to be washed by water shower and be dried by an air flow. The dried vegetal is crushed by a hammer mill until a size inferior to 3 cm.

The washing process of the crushed vegetal material is performed in diffusion containers or in stirred tanks with water between room temperature and 60 °C. The vegetal material ratio versus water is between 1:4 and 1:10 (w/w). As an alternative the washing is performed with a mixture of water and a C₁-C₃ alcohol between 20:80 and 5:95 (v/v) at room temperature. The alcohol is preferably methanol or ethanol and more preferably methanol and the vegetal material ratio versus the mixture of water and methanol is 1:4 (w/w). The washing can also be performed with a mixture of water and acetone between 20:80 and 40:60 (v/v) at room temperature. In a particular embodiment, the ratio water:acetone is 30:70 (v/v) and the vegetal material ratio versus the mixture of water and acetone is between 1:2 and 1:3 (w/w).

The solid and the liquid resulting from the wash are separated by centrifugation, filtration or decantation, repeating the process from the resulting solid as many times as needed to get the washing liquid sweeps along less than 50 g of material per liter of washing liquid, and preferably less than 10 g of material per liter of washing liquid.

In case of vegetal products that are very rich in celluloses it is advisable to use a previous operation of fermentation, in order to increase the yield of the extraction. The vegetal residue, from which water-soluble compounds have already been eliminated, is submerged in a tank with water at a temperature of 37 °C (between 1 and 3 kg of water per kg of wet residue). In this tank with ' water the residue is added, pH is rebalanced to 5 and an enzyme is added, in particular beta-glucosidase (cellulase), between 1 and 25 g per kg of wet residue, depending on the desired magnitude of cellulose hydrolysis, and it is maintained stirred at 37 °C between 2 and 4 hours. Lately, the content of the hydrolysis bath is decanted or centrifuged, the supernatant with sugars from the hydrolysis of celluloses is discarded and the residue is washed with water at 50 °C (4 kg of water per kg of wet residue), it is centrifuged again and the supernatant is discarded.

From the obtained solid at the washing step, whatever using the intermediate fermentation process or not, an extraction is performed in diffusing containers or in stirred tanks. The extraction can be performed with water between 80 °C and its boiling point at conditions of room or pressurized pressure. The ratio of solid versus water is between 1:2 and 1:3 (p/p). As an alternative, the extraction is performed with a C₁-C₃ alcohol at a temperature between 40 °C and its boiling point, preferably methanol between 40 °C and its boiling point, and with a ratio solid:methanol between 1:4 and 1:6 (p/p). The extraction can also be performed with a mixture of water and a glycol, preferably propylene glycol, at a temperature between 80 °C and its boiling point. Finally, the extraction can also be performed with dimethylformamide between 80 °C and its boiling point or dimethyl sulphoxide between 80-150 °C. In a particular embodiment of the invention, the extraction lasts from 1 to 4 hours, and more preferably between 3 and 4 hours. Solid and liquid resulting from the extraction are separated, keeping a temperature superior to 40 °C, by any suitable method, as decantation, filtration or centrifugation.

Next, the dark liquid obtained in the previous step is gone cold usually in decantation tanks. The standing time can vary between 4 and 10 hours. During the process a dark brown precipitate is formed that corresponds to the fraction of the target proanthocyanidines contained in the vegetal product. In case that no precipitate is formed, it is convenient to concentrate the obtained extract by solvent evaporation at low temperature with a reduced pressure at conditions of 50 or 60 °C and from 0.8 to 0.9 kg of absolute pressure. On the contrary, in case of using universal solvents as DMSO, DMF or glycols it is convenient to dilute them with cold water or with an alcohol such as ethanol, propanol, iso-propanol or similar, what allows the precipitation of the low-soluble condensed proanthocyanidines. The resulting precipitate is collected by common methods as decantation, filtration or centrifugation.

The precipitate obtained in the previous step is dried, preferably by hot air flow over plates until a solvent content inferior to a 10% by weight. In a particular embodiment, the precipitate is dried at 80-90 °C. Next, the obtained product is undergone to 120-180 °C at least for 3 minutes. Thus, eliminating the water previously, it is achieved a controlled and homogeneous increase of the temperature of the product and a high polymerization at a very high temperature without risk of overheating in dehydrated zones that occurs at longer polymerization treatments with wet product. Thus, is increased the polymerization rate versus other types of denaturization treatments.

Finally the product is chilled. A final product is obtained with vitreous aspect, brown-color, and which is easily broken. Once cold, it is milled by hammer mills with a mesh inferior to 0.5 mm until getting a size inferior to 180 µm. It is passed through a sieve with a mesh of 180 µm and the brown colored product is recovered.

By the procedure of the invention between 1 and 20 g of product of vegetal origin per kg of dry processed vegetal are obtained, depending on the agronomic variety and on the conditions of the extraction.

In another aspect, the invention provides a pharmaceutical composition comprising a therapeutically effective amount of the product of vegetal origin defined above, together with pharmaceutically acceptable excipients. The invention also provides a dietary complement comprising an effective amount of the product of vegetal origin defined above, together with appropriate amounts of excipients. Moreover a food product is provided which comprises a nutraceutically effective amount of the product of vegetal origin as defined above together with appropriate amounts of other edible ingredients.

The most common presentation forms for the pharmaceutical product and the dietary complement are suspensions, capsules, tablets, powder or pellets but are not excluded other forms that the person skilled in the art considers viable for the administration of the product of the invention. The food product can be, intending not to act as exclusion, as cookies or similar, or in spreadable cream form.

In a last aspect, the invention is related to the use of the product of vegetal origin for the preparation of a medicament, a pharmaceutical composition, a dietary complement or a food product for the treatment and/or the prevention of the hypercholesterolaemia and/or cardiovascular diseases in a mammal, including a human.

Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples are provided by way of illustration, and are not intended to be limiting of the present invention.

### DETAILED DESCRIPTION OF PARTICULAR EMBODIMENTS

### Preparation process starting with pomegranate

It is started with 1000 kg of milled pulp of pomegranate fruit. The content in humidity should not be superior to 10% (w/w) and the size should be inferior to 3 cm. The milled pulp is treated with water at room temperature at 1:10 ratio for 4 hours. The washing water is eliminated with the solved products passing it through a 100 µm sieve and the entire solid is collected and put together with the rest of the solids. The previous washing process is repeated two more times in order to eliminate the water-soluble molecules. The temperature of the last wash is increased until 60 °C for a better extraction of the remains of soluble products. It is advisable to be careful of collecting all the solids that remain in the 100 µm sieve. The solid residue is treated in a tank with reflux, with a mixture of propylene glycol and water in a proportion of 75:25 (v/v) at 95 °C for 4 hours to dissolve the proanthocyanidines. The hot liquid (> 80 °C) is filtered or centrifuged to obtain a dark brown solution which contains the proanthocyanidines. It is concentrated under vacuum at a temperature near to 60 °C until water is eliminated. The obtained liquid is gone cold until room temperature (a minimum of 24 hours). The brown precipitate is recovered by centrifugation and a wet brown-colored paste is obtained. The previous liquid residue is diluted with cold water in a dilution of 1 volume of liquid residue with 9 volumes of water. It is left during 24 hours and the formed solid is recovered by centrifugation. It is put together with the solid obtained in the previous step. The obtained creamy paste is spreaded over plates forming layers not thicker than 0.5 cm and it is left in a drying-oven at low temperature, not superior to 80-90 °C, until the humidity is reduced to a 10%. The oven temperature is increased to 150 °C and it is maintained for 15 min. The obtained product has the dark-brown vitreous appearance. It is milled to a particle size inferior to 170 µm, and it is passed through a 100 µm sieve. The yield is 1-4% from the milled pomegranate fruit.

### Preparation process starting with carob bean

It is started with carob bean pulp, clean, dried and without seed. An amount of 100 kg of carob bean pulp is crushed passing it once through a hammer mill until a size inferior to 3 cm. The crushed product is put in stirred tanks, in 400 kg of a mixture water:metanol 20:80 (v/v) at room temperature during 2 hours. The washing liquid is discarded repeating the process some times to get an elimination of the product of less than 10 g per liter. Later, the solid phase is separated by decantation. The diffusion treatment at room temperature with a mixture of water:acetone 30:70 (v/v) (2 kg of water per kg of wet residue) is repeated with the solid phase during 3 hours. Later, the extraction liquid is separated by filtration and discarded. The residue is evaporated of solvent in vacuum heaters, spreading it over plates at a thickness between 1 and 1.5 cm and heating it at 45 °C during 5 hours. The obtained solid is subjected to a stirred diffusion treatment with water (2 kg of water per kg of wet residue), at 100 °C during 3 hours. Later, the extracting liquid is separated from the insoluble material by hot filtration and the solid residue is discarded. This liquid is gone cold in decantation tanks to get a temperature between 15 and 20°C, maintaining it in such conditions during 6 hours. The precipitate is collected by decantation or centrifugation. The collected amount varies between 0.2 and 2 kg of dry weight depending on the condition of the fruit. The evaporation of the water from the residue is performed spreading it over plates with a product thickness between 1 and 1.5 cm, and drying it in heater during 12 hours at 0.2 atmospheres of pressure and 85°C, until a content of water inferior to 10%. Finally, when the residue does not content more than 10% of water, the dried product is subjected to a temperature of 180 °C in an air flow for 3 minutes, followed by chilling at room temperature. In this way it is obtained a product, that when cold, it is milled in hammer mills with a mesh inferior to 0.5 mm until a size inferior to 180 µm. It is passed through a sieve with a net size of 180 µm and the finished product is obtained.

### Effect of the product of vegetal origin in cholesterolaemia

Influence over cholesterolaemia in rats was studied with three types of dietary fiber and the product of vegetal origin of the invention: microcrystalline cellulose (CEL) AVICEL^{®}, carob fiber (CF) from C.G.A. S.A., modified polyphenols (MP) according to the patent document ES 2.187.356 and the product of vegetal origin (PVO) of the present invention. The fibers were included into four semi-synthetic isocaloric diets adjusted to the nutritional needs of the rats. The base-diet used was AIN 93-G # 110113 Purified Diet (Dyets Inc., Pennsylvania), and the only variable was the type of food fiber, always in a proportion of 5% of the diet. "Wistar" male rats were used, from the Servicio de Animales del Centro Mixto Departamento de Nutrición y Bromatologia I and from Instituto de Nutrición CSIC-UCM (Facultad de Farmacia U.C.M.), selected from the same litter. 40 young rats were used to develop an experimental cholesterolaemia provoked by the consumption during 15 days cholesterol 8 g/kg of diet and bovine bile 2 g/kg of diet, both from Farmitalia-Carlo Erba, Madrid, together with their maintaining diet. During the experimental period the animals were placed in individual metabolism cells. The metabolism cells were kept in a room at 22 ± 2°C, with an automatic light-darkness control (12:12 hours) and constant air circulation.

Rats were divided in 4 groups of 10 hypercholesterolaemiac rats (serum cholesterol 250 ± 37 mg/dl) which, in the same conditions, consumed the cholesterol supplemented diet for 20 days more, but eating also each of the essayed fibers at the level of 5%. During the experimental period of 20 days the animals were fed following the same protocol. The initial serum cholesterol was determined extracting blood from the tail vein of the animal by puncture, after a vasodilatation in a bath at 37°C. Blood was collected in a capillary tube treated with heparin, and cholesterol was quantified immediately by an automatic analyzer. Cholesterol after the 20 days was determined in serum by cannulating the jugular vein, after anaesthetizing the animals.

It was found (cf. TABLE 1) that cellulose did not take effect in cholesterolaemia but serum cholesterol continued to increase in the animals which had consumed the cellulose. On the contrary, the rest of the fibers reduced cholesterol from the initial value. Nevertheless, PVO produced a superior reduction of the serum cholesterol values of the animals. Thus, insoluble polyphenols of PVO have a much superior cholesterol reduction effect than the CF, and superior also to PM, product which has the superior effect over cholesterolaemia described so far in a food fiber (cf. L. Pérez-Olleros et al., J Sci Food Agric 1999, vol. 79, pp. 173-8; H.J. Zunft et al., Adv Ther 2001, vol. 18, pp. 230-6; B. Ruiz-Roso et al., Schironia, vol. 2, pp. 5-9). This effect seems to be produced by the kidnapping of biliary salts by the insoluble polyphenols, with the result of the break of the cholesterol enterohepatic cycle.

TABLE 1 shows the evolution of cholesterolaemia of rats with experimental hypercholesterolaemia which took diets including microcrystalline cellulose (CEL), carob fiber (CF), modified polyphenols (MP) and the product of vegetal origin of the invention (PVO). An (a) indicates that differs significantly p < 0.05 from CEL lot; a (b) indicates that differs significantly p < 0.05 from basal hypercholesterolaemia and, a (c) indicates that differs significantly p < 0.05 from MP lot.

| | serum cholesterol (mg/dl) | serum cholesterol (mg/dl) |
|---|---|---|
| | day 0 | day 20 |
| PVO | 250 ± 37 | 125 ± 18 ^{abc} |
| CEL | 250 ± 37 | 275 ± 25 |
| MP | 250 ± 37 | 140 ± 10 ^{ab} |
| CF | 250 ± 37 | 180 ± 20 ^{ab} |

## Claims

1. A preparation process of a product of vegetal origin, which comprises the steps of:
(a) performing a wash of the crushed vegetal material with a liquid selected from the group consisting of water between room temperature and 60 °C; a mixture of water and a C₁-C₃ alcohol between 20:80 and 5:95 (v/v) at room temperature; and a mixture of water and acetone between 20:80 and 40:60 (v/v) at room temperature; and separating the solid and the liquid resulting from the wash as many times as needed to get the washing liquid sweeping along less than 50 g of material per liter of washing liquid;
(b) performing an extraction from the solid obtained in step (a) at room or pressurized pressure, in conditions selected from the group consisting of water between 80 °C and its boiling point; a C₁-C₃ alcohol between 40 °C and its boiling point; a mixture of water and a glycol between 80 °C and its boiling , point; dimethylformamide between 80°C and its boiling point; and dimethylsulphoxide between 80-150 °C, and separating the solid and the liquid resulting from the extraction keeping a temperature higher than 40°C;
(c) chilling the liquid obtained in step (b) and collecting the resulting precipitate, optionally evaporating part of the solvent before chilling;
(d) drying the precipitate obtained in step (c) until a solvent content lower than 10% percent by weight;
(e) subjecting the product obtained in step (d) to 120-180 °C for at least 3 minutes; and
(f) chilling the product obtained in step (e).

2. The process according to claim 1, wherein the selected liquid in step (a) is water between room temperature and 60°C and the vegetal material ratio versus water is between 1:4 and 1:10 (w/w).

3. The process according to claim 1, wherein the selected liquid in step (a) is a mixture of water and methanol between 20:80 and 5:95 (v/v) at room temperature and the vegetal material ratio versus the mixture of water and methanol is 1:4 (w/w).

4. The process according to claim 1, wherein the selected liquid in step (a) is a mixture of water and acetone 30:70 (v/v) at room temperature and the vegetal material ratio versus the mixture of water and acetone is between 1:2 and 1:3 (w/w).

5. The process according to claim 1, wherein the selected liquid in step (b) is water at a temperature between 80°C and its boiling point and the ratio of solid obtained in step (a) versus the water added is between 1:2 and 1:3 (w/w).

6. The process according to any of the claims 1-4, wherein the selected liquid in step (b) is methanol between 40°C and its boiling point and the ratio of solid obtained in step (a) versus the liquid is between 1:4 and 1:6 (w/w).

7. The process according to any of the claims 1-4, wherein in step (b) the extraction lasts from 1 to 4 hours.

8. The process according to any of the claims 1-7, wherein in step (d) the precipitate is dried at 80-90 °C.

9. The process according to any of the claims 1-8, wherein between step (a) and step (b) a fermentation of the material is performed.

10. A product of vegetal origin obtainable by the process according to any of the claims 1-9.

11. The product of vegetal origin according to claim 10, which is obtained from dicotyledons.

12. The product of vegetal origin according to claim 11, which is obtained from pomegranate tree fruit.

13. The product of vegetal origin according to claim 11, which is obtained from carob bean pulp.

14. A pharmaceutical composition comprising a therapeutically effective amount of the product of vegetal origin as defined in any of the claims 10-13, together with pharmaceutically acceptable excipients.

15. A dietary complement comprising the product of vegetal origin as defined in any of the claim 10-13, together with appropriate amounts of excipients.

16. A food product comprising a nutraceutically effective amount of the product of vegetal origin as defined in any of the claims 10-13, together with appropriate amounts of other edible ingredients.

17. Use of the product of vegetal origin as defined in any of the claims 10-13, for the preparation of a medicament for the treatment and/or the prevention of hypercholesterolaemia and/or cardiovascular diseases in a mammal, including a human.

18. Use of the product of vegetal origin as defined in any of the claims 10-13, for the preparation of a dietary complement for the treatment and/or the prevention of hypercholesterolaemia and/or cardiovascular diseases in a mammal, including a human.

19. Use of the product of vegetal origin as defined in any of the claims 10-13, for the preparation of a food product for the treatment and/or the prevention of hypercholesterolaemia and/or cardiovascular diseases in a mammal, including a human.

## Patentansprüche

1. Ein Verfahren zur Herstellung eines Produkts pflanzlicher Herkunft, welches die folgenden Schritte umfasst:
a) Durchführung einer Wässerung von zerstoßenem pflanzlichen Material mit einer Flüssigkeit, die aus einer Gruppe ausgewählt ist, die aus Wasser zwischen Raumtemperatur und 60°C; eine Mischung aus Wasser und einem C₁₋C₃-Alkohol in einem Verhältnis von zwischen 20:80 und 5:95 (V/V) bei Raumtemperatur; und eine Mischung aus Wasser und Aceton in einem Verhältnis von zwischen 20:80 und 40:60 (V/V) bei Raumtemperatur besteht; und die Durchführung der Trennung des festen von dem flüssigen Anteil, welche sich aus der Wässerung ergeben, so viele Male wie nötig sind, um eine Wässerungsflüssigkeit zu bekommen, die weniger als 50 g des Materials pro Liter der Wässerungsflüssigkeit mitreißt;
b) Durchführung einer Extraktion von dem festen Bestandteil, den man bei Schritt (a) erhält, bei Raumdruck oder bei Überdruck, unter Bedingungen, ausgewählt aus einer Gruppe, die aus Wasser zwischen 80°C und dessen Siedepunkt; ein C₁-C₃-Alkohol zwischen 40°C und dessen Siedepunkt; eine Mischung aus Wasser und einem Glykol zwischen 80°C und dessen Siedepunkt; Dimethylformamid zwischen 80°C und dessen Siedepunkt; und Dimethylsulfoxid zwischen 80-150 °C besteht, und die Durchführung der Trennung des festen von dem flüssigen Anteil, welche sich aus der Extraktion ergeben, wobei eine Temperatur aufrecht erhalten wird, die über 40°C liegt;
c) Abkühlen der Flüssigkeit, die man bei Schritt (b) erhält und das Auffangen des sich daraus ergebenden Präzipitat, optional die Verdampfung eines Teils des Lösemittels vor dem Abkühlen;
d) Trocknen des Präzipitats, das man bei Schritt (c) erhält, bis zu einem Lösemittelgehalt, der unter 10 Gew.-% liegt.
e) Aussetzen des Produkts, das man bei Schritt (d) erhält, bei 120-18°C für mindestens 3 Minuten; und
f) Abkühlen des Produkts, das man bei Schritt (e) erhält.

2. Das Verfahren nach Anspruch 1, wobei die bei Schritt (a) ausgewählte Flüssigkeit Wasser zwischen Raumtemperatur und 60°C ist, und das Verhältnis zwischen pflanzlichem Material und dem Wasser bei zwischen 1:4 und 1:10 (G/G) liegt.

3. Das Verfahren nach Anspruch 1, wobei die bei Schritt (a) ausgewählte Flüssigkeit eine Mischung aus Wasser und Methanol in einem Verhältnis von zwischen 20:80 und 5:95 (V/V) bei Raumtemperatur ist, und das Verhältnis zwischen dem pflanzlichen Material und der Mischung aus Wasser und Methanol bei 1:4 (G/G) liegt.

4. Das Verfahren nach Anspruch 1, wobei die bei Schritt (a) ausgewählte Flüssigkeit eine Mischung aus Wasser und Aceton bei einem Verhältnis von 30:70 (V/V) bei Raumtemperatur ist, und das Verhältnis zwischen dem pflanzlichen Material und der Mischung aus Wasser und Aceton bei zwischen 1:2 und 1:3 (G/G) liegt.

5. Das Verfahren nach Anspruch 1, wobei die bei Schritt (b) ausgewählte Flüssigkeit Wasser bei einer Temperatur von zwischen 80°C und dessen Siedepunkt ist, und das Verhältnis zwischen dem festen Bestandteil, den man bei Schritt (a) erhält, und dem hinzugefügten Wasser bei zwischen 1:2 und 1:3 (G/G) liegt.

6. Das Verfahren nach einem der Ansprüche 1-4, wobei di bei Schritt (b) ausgewählt Flüssigkeit Methanol bei einer Temperatur von zwischen 40°C und dessen Siedepunkt ist, und das Verhältnis zwischen dem festen Bestandteil, den man bei Schritt (a) erhält, und der Flüssigkeit bei zwischen 1:4 und 1:6 (G/G) liegt.

7. Das Verfahren nach einem der Ansprüche 1-4, wobei die Extraktion bei Schritt (b) zwischen 1 und 4 Stunden dauert.

8. Das Verfahren nach einem der Ansprüche 1-7, wobei das Präzipitat bei 80-90°C getrocknet wird.

9. Das Verfahren nach einem der Ansprüche 1-8, wobei zwischen Schritt (a) und Schritt (b) eine Fermentierung des Materials durchgeführt wird.

10. Ein Produkt pflanzlicher Herkunft, welches durch das Verfahren nach einem der Ansprüche 1-9 erhältlich ist.

11. Das Produkt pflanzlicher Herkunft nach Anspruch 10, welches aus Dikotyledonen erhalten wird.

12. Das Produkt pflanzlicher Herkunft nach Anspruch 11, welches aus Granatäpfeln erhalten wird.

13. Das Produkt pflanzlicher Herkunft nach Anspruch 11, welches aus Johannisbrotschotenpulpe erhalten wird.

14. Eine pharmazeutische Verbindung, die eine therapeutisch wirksame Menge des Produktes pflanzlicher Herkunft, so wie in einem der Ansprüche 10-13 definiert, zusammen mit geeigneten Mengen an Arzneistoffträgern umfasst.

15. Eine Ernährungsergänzung, die das Produkt pflanzlicher Herkunft, so wie in einem der Ansprüche 10-13 definiert, zusammen mit geeigneten Mengen an Hilfstoffe umfasst.

16. Ein Nahrungsmittelprodukt, das eine nutrazeutisch wirksame Menge des Produktes pflanzlicher Herkunft, so wie in einem der Ansprüche 10-13 definiert, zusammen mit geeigneten Mengen anderer essbarer Zutaten umfasst.

17. Die Verwendung des Produktes pflanzlicher Herkunft, so wie in einem der Ansprüche 10-13 definiert, zur Herstellung eines Medikaments zur Behandlung und/oder zur Prävention von Hypercholesterinämie und/oder kardiovaskulären Krankheiten bei einem Säugetier, wobei Menschen eingeschlossen sind.

18. Die Verwendung des Produktes pflanzlicher Herkunft, so wie in einem der Ansprüche 10-13 definiert, zur Herstellung einer Ernährungsergänzung zur Behandlung und/oder zur Prävention von Hypercholesterinämie und/oder kardiovaskulären Krankheiten bei einem Säugetier, wobei Menschen eingeschlossen sind.

19. Die Verwendung des Produktes pflanzlicher Herkunft, so wie in einem der Ansprüche 10-13 definiert, zur Herstellung eines Nahrungsmittelprodukts zur Behandlung und/oder zur Prävention von Hypercholesterinämie und/oder kardiovaskulären Krankheiten bei einem Säugetier, wobei Menschen eingeschlossen sind.

## Revendications

1. Un procédé de préparation d'un produit d'origine végétale, qui comprend les étapes consistant à:
(a) procéder à un lavage de la matière végétale broyée avec un liquide choisi dans le groupe composé d'eau entre température ambiante et 60°C; d'un mélange d'eau et d'un C₁-C₃ alcool entre 20:80 et 5:95 (v/v) à température ambiante; et d'un mélange d'eau et d'acétone entre 20:80 et 40:60 (v/v) à température ambiante; et séparer le solide et le liquide résultant du lavage autant de fois qu'il sera nécessaire pour que le liquide de lavage entraîne moins de 50 g de matière par litre de liquide de lavage;
(b) réaliser une extraction à partir du solide obtenu au cours de l'étape (a) à pression ambiante ou sous pression, dans des conditions choisies dans le groupe composé d'eau entre 80°C et son point d'ébullition; d'un C₁-C₃ alcool entre 40°C et son point d'ébullition; d'un mélange d'eau et d'un glycol entre 80°C et son point d'ébullition; de diméthylformamide entre 80°C et son point d'ébullition; et de diméthylsulfoxyde entre 80-150°C, et séparer le solide et le liquide résultant de l'extraction en maintenant une température supérieure à 40°C;
(c) refroidir le liquide obtenu au cours de l'étape (b) et recueillir le précipité en résultant, éventuellement en faisant s'évaporer une partie du solvant avant le refroidissement;
(d) sécher le précipité obtenu au cours de l'étape (c) jusqu'à une teneur en solvant inférieure à 10% en poids;
(e) soumettre le produit obtenu au cours de l'étape (d) à 120-180°C pendant au moins trois minutes; et
(f) refroidir le produit obtenu au cours de l'étape (e).

2. Le procédé selon la revendication 1, dans lequel le liquide sélectionné au cours de l'étape (a) est de l'eau entre température ambiante et 60°C et dans lequel la proportion de matière végétale par rapport à l'eau est entre 1:4 et 1:10 (m/m).

3. Le procédé selon la revendication 1, dans lequel le liquide sélectionné au cours de l'étape (a) est un mélange d'eau et de méthanol entre 20:80 et 5:95 (v/v) à température ambiante et dans lequel la proportion de matière végétale par rapport au mélange d'eau et de méthanol est de 1:4 (m/m).

4. Le procédé selon la revendication 1, dans lequel le liquide sélectionné au cours de l'étape (a) est un mélange d'eau et d'acétone 30:70 (v/v) à température ambiante et dans lequel la proportion de matière végétale par rapport au mélange d'eau et d'acétone est entre 1:2 et 1:3 (m/m).

5. Le procédé selon la revendication 1, dans lequel le liquide sélectionné au cours de l'étape (b) est de l'eau à une température entre 80°C et son point d'ébullition et dans lequel la proportion de solide obtenu au cours de l'étape (a) par rapport à l'eau ajoutée est entre 1:2 et 1:3 (m/m).

6. Le procédé selon l'un quelconque des revendications 1-4, dans lequel le liquide sélectionné au cours de l'étape (b) est du méthanol entre 40°C et son point d'ébullition et dans lequel la proportion de solide obtenu au cours de l'étape (a) par rapport au liquide est entre 1:4 et 1:6 (m/m).

7. Le procédé selon l'un quelconque des revendications 1-4, dans lequel au cours de l'étape (b), l'extraction dure de 1 à 4 heures.

8. Le procédé selon l'un quelconque des revendications 1-7, dans lequel au cours de l'étape (d), le précipité est séché à 80-90°C.

9. Le procédé selon l'un quelconque des revendications 1-8, dans lequel entre l'étape (a) et l'étape (b) on procède à une fermentation de la matière.

10. Un produit d'origine végétale pouvant être obtenu par le procédé selon l'un quelconque des revendications 1-9.

11. Le produit d'origine végétale selon la revendication 10, qui est obtenu à partir de dicotylédones.

12. Le produit d'origine végétale selon la revendication 11, qui est obtenu à partir du fruit du grenadier.

13. Le produit d'origine végétale selon la revendication 11, qui est obtenu à partir de pulpe de caroube.

14. Une composition pharmaceutique comprenant une quantité thérapeutiquement efficace du produit d'origine végétale tel qu'il est défini dans l'une quelconque des revendications 10-13, conjointement avec des excipients pharmaceutiquement acceptables.

15. Un complément alimentaire comprenant le produit d'origine végétale tel qu'il est défini dans l'une quelconque des revendications 10-13, conjointement avec des quantités adaptées d'excipients.

16. Un produit alimentaire comprenant une quantité nutriceutiquement efficace du produit d'origine végétale tel qu'il est défini dans l'une quelconque des revendications 10-13, conjointement avec des quantités adaptées d'autres ingrédients comestibles.

17. Utilisation du produit d'origine végétale tel qu'il est défini dans l'une quelconque des revendications 10-13, pour la préparation d'un médicament pour le traitement et/ou la prévention de l'hypercholestérolémie et/ou de maladies cardiovasculaires chez un mammifère, y compris un être humain.

18. Utilisation du produit d'origine végétale tel qu'il est défini dans l'une quelconque des revendications 10-13, pour la préparation d'un complément alimentaire pour le traitement et/ou la prévention de l'hypercholestérolémie et/ou de maladies cardiovasculaires chez un mammifère, y compris un être humain.

19. Utilisation du produit d'origine végétale tel qu'il est défini dans l'une quelconque des revendications 10-13, pour la préparation d'un produit alimentaire pour le traitement et/ou la prévention de l'hypercholestérolémie et/ou de maladies cardiovasculaires chez un mammifère, y compris un être humain.
